# EUROPEAN PATENT APPLICATION

(11) **EP 3 187 875 A1**
(43) Date of publication of application: **05.07.2017**
(21) Application number: 15846989.0
(22) Date of filing: 17.08.2015
(51) Int. Cl.: G01N 33/543, G01N 21/78, G01N 33/483

(54) **KIT FOR MEASURING TARGET SUBSTANCE, SYSTEM FOR MEASURING TARGET SUBSTANCE, IMMUNOCHROMATOGRAPHIC MEASURING KIT AND IMMUNOCHROMATOGRAPHIC MEASURING SYSTEM**

(30) Priority: 02.10.2014 JP 2014203729
(71) Applicant: Sony Corporation, Tokyo 108-0075 (JP)
(72) Inventor: MATSUMOTO Masahiro, Tokyo 108-0075 (JP)
(74) Representative: D Young & Co LLP
(86) International application number: PCT/JP2015/072989
(87) International publication number: WO 2016/051974

(57) **Abstract**

Provided is a target substance measurement kit for detecting a target substance sensitively in a convenient manner.

Provided is a target substance measurement kit including a capturing unit that captures a target substance contained in a liquid and a flow rate control unit that controls the rate of flow of the liquid in the capturing unit. The flow rate control unit is provided downstream from the capturing unit in the flow direction of the liquid.

## Description

### TECHNICAL FIELD

The present technology relates to a target substance measurement kit, a target substance measurement system, an immunochromatography measurement kit, and an immunochromatography measurement system.

### BACKGROUND ART

As a means for detecting a target substance such as a specific antigen or antibody, an immunoassay method utilizing a specific reaction between an antigen and an antibody against the antigen has been used. Among such methods, "immunochromatography" is a method in which a complex of a substance to be detected contained in a sample and a labeled antibody is formed, then the complex and an antibody that has not bound to the analyte are separated using the principles of chromatography, and the state of aggregation of the complex is measured. Therefore, "immunochromatography" is a method that also allows for visual judgment, and thus has been generally used as a method for detecting a target substance in a convenient manner. For such immunochromatography, the improvement of sensitivity in the detection of an analyte has been required.

Thus, for example, Patent Document 1 discloses "an immunochromatography measurement method for detecting the presence of a substance to be detected in a liquid specimen, in which: on a chromatography media partially having a test region having imparted thereto a first binding substance that specifically binds to the substance to be detected, the test region is impregnated with the liquid specimen containing a visibly labeled second binding substance that specifically binds to the substance to be detected; at the same time as or after the impregnation of the test region with the liquid specimen, the chromatography media is impregnated with a solution for visual recognition having a refractive index of which refractive index difference Δn from the refractive index of the chromatography media is -0.1 ≤ Δn ≤ = 0.1; and the test region is viewed with the test region being impregnated with the solution for visual recognition". According to this method, the chromatography media is impregnated with a solution for visual recognition having a predetermined refractive index, whereby light scattering caused by the refractive index difference at the interface between the chromatography media and the solution for visual recognition can be suppressed. As a result, the visible region of the chromatography media in the thickness direction expands, and higher detection sensitivity can be obtained in visual judgement.

However, according to the above method, after the labeled analyte is separated using the chromatography media, again, a solvent is added to impregnate the chromatography media. This takes a long period of time and impairs the convenience, which is a characteristic of immunochromatography. Thus, for example, Patent Document 2 discloses "an immunochromatography measurement method for detecting the presence of a substance to be detected in a sample using a labeled substance, including: a first step of, with a reactive site on a chromatography media, on which a first substance that specifically binds to the analyte is immobilized, bringing a detection reagent composed of a second substance that specifically binds to the analyte, which has bound thereto the labeled substance, into contact together with or following the sample; a second step of compressing a support substance at the reactive site to reduce the thickness; and a third step of observing a light emission signal from the labeled substance".

### CITATION LIST

### PATENT DOCUMENT

Patent Document 1: Japanese Patent Application Laid-Open No. 2009-257819
Patent Document 2 : Japanese Patent Application Laid-Open No. 2012-215494

### SUMMARY OF THE INVENTION

### PROBLEMS TO BE SOLVED BY THE INVENTION

The immunochromatography measurement method described in Patent Document 2 makes it possible to sensitively detect an analyte without impairing the convenience. However, according to the above method, the step of reducing the thickness of a support substance is indispensable.

Like this, in the detection of a target substance, further improvement to achieve higher convenience and improved detection sensitivity has been required.

Thus, a main object of the present disclosure is to provide a target substance measurement kit for detecting a target substance sensitively in a convenient manner, etc.

### SOLUTIONS TO PROBLEMS

To solve the above problem, the present disclosure provides a target substance measurement kit including: a capturing unit that captures a target substance contained in a liquid; and a flow rate control unit that controls the rate of flow of the liquid in the capturing unit, the flow rate control unit being provided downstream from the capturing unit in the flow direction of the liquid.

The target substance measurement kit may further include upstream from the capturing unit in the flow direction, a liquid introduction unit into which the liquid is introduced, and the thickness of the capturing unit in the direction transverse to the flow direction may be smaller than the thickness of the liquid introduction unit in the transverse direction.

Further, the target substance measurement kit may further include downstream from the flow rate control unit in the flow direction, a liquid holding unit that absorbs the liquid, and the thickness of the capturing unit in the direction transverse to the flow direction may be smaller than the thickness of the liquid holding unit in the transverse direction.

The thickness of the capturing unit in the direction transverse to the flow direction may be 0.01 mm or more and 0.10 mm or less.

The longitudinal direction of the capturing unit may extend in the flow direction.

The flow rate control unit may include a porous body, and the average pore size of the porous body may be smaller than the average pore size of a portion of the capturing unit through which the liquid flows.

The thickness of the flow rate control unit in the direction transverse to the flow direction may be smaller than the thickness of the capturing unit in the transverse direction.

The flow rate control unit may include a filter having through pores formed therein, and the average pore size of the through pores may be smaller than the average pore size of a portion of the capturing unit through which the liquid flows.

The present disclosure also provides a target substance measurement system including a target substance measurement kit and a target substance measurement device, the target substance measurement kit including: a capturing unit that captures a target substance contained in a liquid; and a flow rate control unit that controls the rate of flow of the liquid in the capturing unit, the flow rate control unit being provided downstream from the capturing unit in the flow direction of the liquid, the target substance measurement device including: a light irradiation unit that irradiates the capturing unit with light; and a light detection unit that detects light emitted from the capturing unit.

The target substance measurement kit may include, upstream from the capturing unit in the flow direction, a liquid introduction unit into which the liquid is introduced, and the target substance measurement device may include a liquid injection mechanism that injects the liquid into the liquid introduction unit.

The present disclosure also provides an immunochromatography measurement kit including: a capturing unit containing an antibody immobilized on a flow path through which a liquid containing a target substance flows; and a flow rate control unit containing a porous body disposed on the flow path, the flow rate control unit being provided downstream from the capturing unit in the flow direction of the liquid.

The present disclosure also provides an immunochromatography measurement system including an immunochromatography measurement kit and a target substance measurement device, the immunochromatography measurement kit including: a capturing unit containing an antibody immobilized on a flow path through which a liquid containing a target substance flows; and a flow rate control unit containing a porous body disposed on the flow path, the flow rate control unit being provided downstream from the capturing unit in the flow direction of the liquid, the target substance measurement device including: a light irradiation unit that irradiates the capturing unit with light; and a light detection unit that detects light emitted from the capturing unit.

### EFFECTS OF THE INVENTION

The present disclosure provides a target substance measurement kit for detecting a target substance sensitively in a convenient manner, etc. Incidentally, the effects described herein are not necessarily limited, and any effect described in the present disclosure may be obtained.

### BRIEF DESCRIPTION OF DRAWINGS

Fig. 1 is a schematic diagram showing a configuration example of a target substance measurement system according to a first embodiment of the present disclosure.
Figs. 2A and 2B are schematic diagrams showing an example of a target substance measurement kit according to the first embodiment. A is a plan view, and B is a cross-sectional view along the line P1-P1 shown in A.
Figs. 3A and 3B are schematic diagrams showing an example of the disposition of a second capturing unit in a target substance measurement kit. A is a plan view, and B is a cross-sectional view along the line P2-P2 shown in A.
Figs. 4A to 4C are schematic diagrams showing a configuration example of a flow rate control unit.
Fig. 5 is a schematic diagram showing the detection of a target substance by a target substance measurement system according to the first embodiment.
Figs. 6A to 6C are schematic diagram showing the relation between the average pore size of a portion of a capturing unit through which a liquid flows and the detection of a target substance.
Figs. 7A and 7B are schematic diagrams showing an example of a target substance measurement kit in a target substance measurement system according to a second embodiment of the present disclosure. A is a plan view, and B is a cross-sectional view along the line P3-P3 shown in A.
Figs. 8A and 8B are schematic diagrams showing an example of a target substance measurement kit in a target substance measurement system according to the second embodiment of the present disclosure. A is a plan view, and B is a cross-sectional view along the line P4-P4 shown in A.
Figs. 9A and 9B are schematic diagrams showing an example of a target substance measurement kit in a target substance measurement system according to the second embodiment of the present disclosure. A is a plan view, and B is a cross-sectional view along the line P5-P5 shown in A.
Figs. 10A and 10B are schematic diagram showing the relation between the thickness of a capturing unit and the detection of a target substance.
Figs. 11A and 11B are schematic diagrams showing an example of a target substance measurement kit in a target substance measurement system according to a third embodiment of the present disclosure. A is a plan view, and B is a cross-sectional view along the line P6-P6 shown in A.
Figs. 12A and 12B are schematic diagrams showing an example of a target substance measurement kit in a target substance measurement system according to the third embodiment of the present disclosure. A is a plan view, and B is a cross-sectional view along the line P7-P7 shown in A.
Fig. 13 is a schematic diagram showing a configuration example of a target substance measurement system according to a fourth embodiment of the present disclosure.

### MODE FOR CARRYING OUT THE INVENTION

Hereinafter, preferred modes for carrying out the present disclosure will be described. Incidentally, the embodiments described below show examples of typical embodiments of the present disclosure, and do not narrow the interpretation of the scope of the present disclosure. In addition, the description will be given in the following order.

### 1. First Embodiment

(Example in which a flow rate control unit is provided in a target substance measurement kit)

### 2. Second Embodiment

(Example in which a capturing unit of a target substance measurement kit is formed thinner compared with other components)

### 3. Third Embodiment

(Example in which the longitudinal direction of a capturing unit of a target substance measurement kit extends in the flow direction of a liquid)

### 4. Fourth Embodiment

(Example in which a target substance measurement device includes a liquid injection mechanism)

### 1. First Embodiment

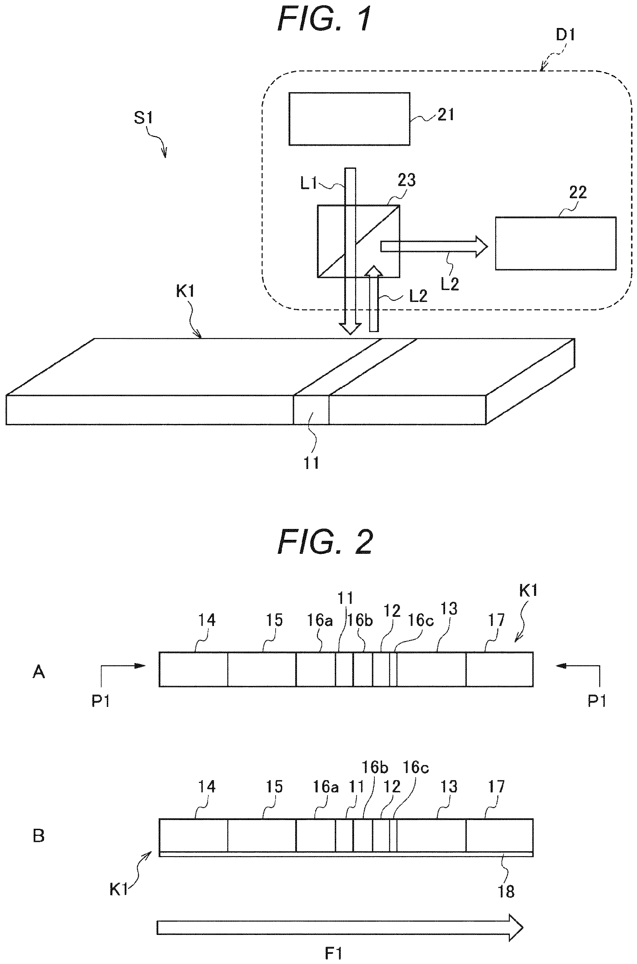

Fig. 1 is a schematic diagram showing a configuration example of a target substance measurement system according to a first embodiment of the present disclosure. In the figure, the target substance measurement system indicated with the reference numeral S1 is roughly composed of a target substance measurement device D1 and a target substance measurement kit K1. The components of the target substance measurement system S1 will be described one by one.

### (1) Target Substance Measurement Kit

First, the target substance measurement kit K1 will be described. Figs. 2A and 2B are schematic diagrams showing a configuration example of the target substance measurement kit K1 according to this embodiment. In addition, Fig. 2A shows a plan view of the target substance measurement kit K1, and Fig. 2B shows a cross-sectional view along the line P1-P1 shown in A. As shown in Fig. 2A, the target substance measurement kit K1 at least includes a capturing unit 11 and a flow rate control unit 13.

In addition, the target substance measurement kit K1 may include components necessary for the detection of a target substance, which is a detection object, by immunochromatography or the like, for example. The target substance measurement kit K1 may also include, for example, as shown in Fig. 2A, a liquid introduction unit 14, a reagent holding unit 15, development units 16a, 16b, and 16c, a second capturing unit 12, and a liquid holding unit 17. In addition, these components may also be fixed to a substrate 18.

As shown in Fig. 2A and Fig. 2B, the liquid introduction unit 14, the reagent holding unit 15, the development units 16a, 16b, and 16c, the capturing unit 11, the second capturing unit 12, the flow rate control unit 13, and the liquid holding unit 17 are each disposed in contact with adjacent other units. Accordingly, due to a capillary phenomenon, a liquid introduced into the liquid introduction unit 14 described below flows in the direction shown by the arrow F1 and can reach the liquid holding unit 17 (see Figs. 2A and 2B, arrow F1). Incidentally, in the present disclosure, unless otherwise noted, the liquid introduction unit 14 side is the upstream side, and the liquid holding unit 17 side is the downstream side.

### <Liquid Introduction Unit>

The liquid introduction unit 14 is a component for a liquid to be introduced into the target substance measurement kit K1. In addition, the liquid introduction unit 14 separates a target substance and substances other than the target substance contained in the liquid and disperses the target substance. As long as such a function is achieved, the liquid introduction unit 14 maybe freely selected from known materials. For example, the liquid introduction unit 14 can be employed from several porous bodies, such as a cellulose membrane, a nitrocellulose membrane, an acetyl cellulose membrane, a polysulfone membrane, a polyethersulfone membrane, a nylon membrane, glass fibers, and a nonwoven fabric. In addition, in order to suppress nonspecific binding to a target substance, it is preferable that the liquid introduction unit 14 is treated with a blocking agent such as albumin.

In the present disclosure, the "liquid" to be introduced into the liquid introduction unit 14 should be a liquid object containing a target substance that can be detected by immunochromatography or the like, for example, and its composition is not particularly limited. Examples of liquids includes biologically derived samples and liquid samples prepared by adding a reagent to such a sample or diluting the sample. Examples of biologically derived samples include blood, plasma, serum, cerebrospinal fluid, saliva, semen, urine, nasal swabs, and throat swabs.

In addition, the kind of target substance is not particularly limited as long as it can be detected by immunochromatography or the like, for example. Examples of target substances include proteins, peptides, nucleic acids, saccharides, glycolipids, and complex carbohydrates.

### <Reagent Holding Unit>

The reagent holding unit 15 is a region in which a detection reagent that binds to a target substance is held. As long as a detection reagent is held in the reagent holding unit 15 in such a manner that it can bind to a target substance in a liquid introduced into the liquid introduction unit 14, the configuration of the reagent holding unit 15 is not particularly limited. For example, a detection reagent is applied to a porous body such as a cellulose filter paper or a glass-fiber filter paper and dried, and the porous body is used as the reagent holding unit 15, whereby the detection reagent can be held in the reagent holding unit 15 in dry state. When a detection reagent is used in dry state, the detection reagent is dissolved by a liquid flowing into the reagent holding unit 15, whereby a target substance in the liquid and the detection reagent can be brought into contact. In addition, in order to suppress nonspecific binding to a target substance, it is preferable that the reagent holding unit 15 is also treated with a blocking agent such as albumin.

The detection reagent should include a structure for specifically binding to a target substance and a structure to serve as a label in the detection of the target substance, and may be suitably selected from known materials according to the properties of the target substance, etc. As the structure for specifically binding to a target substance, an antibody, an aptamer, a molecular imprinted polymer, or the like may be used. In addition, as labels to be applied to such antibodies, aptamers, molecular imprinted polymers, and the like, gold colloid, fluorescent dyes, complexes such as a ruthenium complex and an iridium complex, organic compounds showing chemiluminescence such as luminol, lucigenin, dioxetane, and oxalate, substances showing bioluminescence such as luciferin, and the like can be mentioned.

### <Development Unit>

The development units 16a, 16b, and 16c are components for separating a complex of a detection reagent and a target substance bound together and a detection reagent that has not bound to a target substance using the principles of chromatography. As long as the separation described above can be performed, the development units 16a, 16b, and 16c may be freely selected from known materials according to the properties of the target substance and the detection reagent, etc. For example, as the development units 16a, 16b, and 16c, membranes made of various materials for use as chromatography media can be employed. Examples of such membranes include a nitrocellulose membrane, a mixed nitrocellulose ester membrane,a polyvinylidenefluoride (PVDF) membrane, a nylon membrane, and a polyethersulfone membrane.

In addition, although the development units 16a, 16b, and 16c are provided in three locations in Fig. 2A and Fig. 2B, the disposition of the development units 16a, 16b, and 16c is not limited to the illustrated disposition. For example, in the target substance measurement kit K1, the second capturing unit 12 and flow rate control unit 13 described below may be disposed in direct contact with each other, and the development unit 16c does not have to be provided. In addition, in the case where the second capturing unit 12 is not provided in the target substance measurement kit K1, the capturing unit 11 and the flow rate control unit 13 may be disposed in direct contact with each other.

### <Capturing Unit>

The capturing unit 11 is a component for capturing a target substance contained in a liquid. As long as the capturing unit 11 can capture a target substance in a liquid, the specific configuration thereof is not limited and may be freely designed from known materials. As the capturing unit 11, for example, a membrane made of nitrocellulose, polyvinylidene fluoride (PVDF), nylon, polyethersulfone, or the like having fixed thereto a substance that specifically binds to the target substance may also be employed. In addition, as substances that specif ically bind to a target substance, antibodies, aptamers, molecular imprinted polymers, and the like can be mentioned, for example. Such a substance that specifically binds to a target substance, such as an antibody, is immobilized on the membrane described above by hydrophobic binding, electrostatic binding, ion binding, or the like.

For example, in the case where the target substance is captured by immunochromatography, the capturing unit 11 employed may be such that an antibody for capturing a target substance is immobilized on a flow path through which a liquid containing the target substance flows.

In addition, in the target substance measurement kit, a plurality of capturing units 11 may also be provided corresponding to the number of kinds of target substances. In this case, in each capturing unit 11, an antibody or the like corresponding to the target substance to be captured is immobilized. Specifically, a substance that specifically binds to a first target substance is immobilized on one capturing unit 11, and a substance that specifically binds to a second target substance is immobilized on another capturing unit 11, whereby a plurality of target substances can be detected in one measurement using the target substance measurement kit K1.

Incidentally, in the present disclosure, the capturing unit 11 described above is conveniently also referred to as "first capturing unit 11" in order to distinguish from the second capturing unit 12 described below.

### <Second Capturing Unit>

The second capturing unit 12 is a component for capturing a detection reagent held in the reagent holding unit 15. After the introduction of a liquid into the liquid introduction unit 14, the detection reagent comes in contact with the liquid in the reagent holding unit 15 and flows through the inside of the target substance measurement kit K1. In the case where the detection reagent is present in excess of a target substance contained in the liquid, a part of the detection reagent cannot bind to the target substance, and thus is not captured by the first capturing unit 11 and further moves toward the downstream side. The second capturing unit 12 captures such a detection reagent.

As long as the second capturing unit 12 can capture the detection reagent in the liquid, the specific configuration thereof is not limited and may be freely designed from known materials. As the second capturing unit 12, for example, similarly to the first capturing unit 11 described above, it is also possible to employ a membrane having immobilized thereon a substance that specifically binds to the detection reagent.

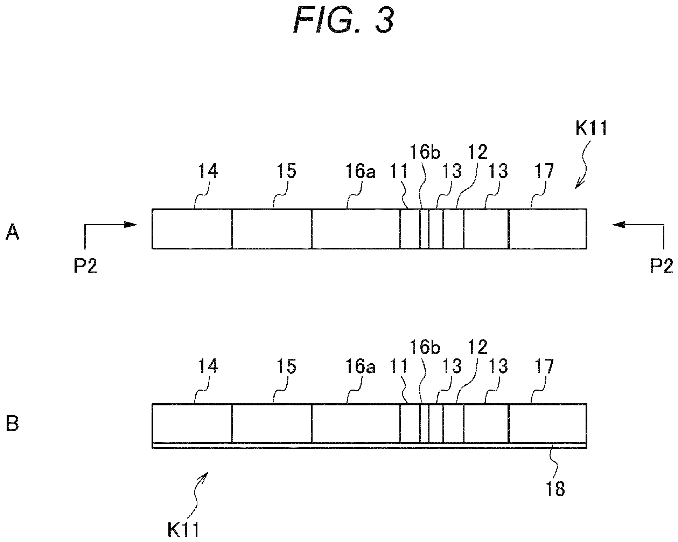

In addition, the position in which the second capturing unit 12 is provided should be downstream from the first capturing unit 11 and upstream from the liquid holding unit 17 described below, and is not limited to the disposition shown in Figs. 2A and 2B. Figs. 3A and 3B are schematic diagrams showing an example of the disposition of the second capturing unit 12. Fig. 3A is a plan view of the target substance measurement kit K11, and Fig. 3B is a cross-sectional view along the line P2-P2 shown in Fig. 3A.

As shown in Figs. 3A and 3B, the second capturing unit 12 may be provided in the flow rate control unit 13. For example, a substance capable of specifically binding to the detection reagent may be immobilized in a predetermined space in the flow rate control unit 13, thereby allowing for the function as a second capturing unit 12. Also in the other embodiments described below, the second capturing unit 12 may be provided similarly to this embodiment.

The second capturing unit 12 is not an indispensable component for the detection of a target substance in the target substance measurement kit K1, but is preferably provided in the target substance measurement kit K1 for the reasons described below. In addition, also in the second embodiment, third embodiment, and fourth embodiment described below, it is preferable that the second capturing unit 12 is included similarly to the first embodiment. In addition, the disposition of the second capturing unit 12 should also be, similarly to this embodiment, downstream from the first capturing unit 11 and upstream from the liquid holding unit 17 described below, and is not particularly limited.

### <Flow Rate Control Unit>

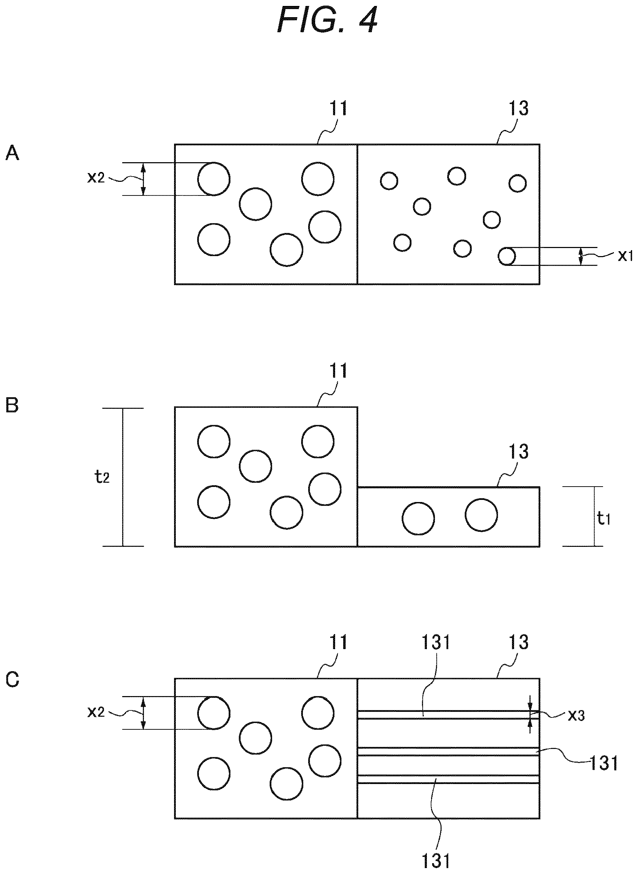

The flow rate control unit 13 is a component for controlling the rate of flow of a liquid in the first capturing unit 11. The flow rate control unit 13 is provided downstream from the first capturing unit 11 in the flow direction of the liquid (see Fig. 2A, arrow F1). As long as the low rate control unit 13 can control the speed of the liquid flowing through the first capturing unit 11, the specific configuration thereof is not particularly limited and may be freely designed from known materials. Figs. 4A to 4C schematically show a configuration example of the flow rate control unit 13.

As shown in Fig. 4A, for example, the flow rate control unit 13 may include a porous body. In addition, in this case, it is preferable that the average pore size of the porous body (see Fig. 4A, x1) is smaller than the average pore size of a portion of the first capturing unit 11 through which the liquid flows (see Fig. 4A, x2). By reducing the average pore size of the porous body provided in the flow rate control unit 13, because of such a flow rate control unit 13, the rate of flow of a liquid in the first capturing unit 11 can be made smaller than in the case where the flow rate control unit 13 is not provided.

For example, in the case where a target substance is captured by immunochromatography, it is preferable that the flow rate control unit 13 including a porous body described above is provided on a flow path through which a liquid containing a target substance flows.

In addition, as shown in Fig. 4B, for example, the thickness of the flow rate control unit 13 in the direction transverse to the flow direction (see Fig. 4B, t1) may be smaller than the thickness of the first capturing unit 11 in the direction transverse to the flow direction (see Fig. 4B, t2). By reducing the thickness of the flow rate control unit 13, because of such a flow rate control unit 13, the rate of flow of a liquid in the first capturing unit 11 can be made smaller than in the case where the flow rate control unit 13 is not provided.

Further, as shown in Fig. 4C, for example, it is possible that the flow rate control unit 13 includes a filter having through pores 131 formed therein, and the average pore size of the through pores 131 (see Fig. 4C, x3) is smaller than the average pore size of a portion of the first capturing unit 11 through which the liquid flows (see Fig. 4C, x2). By reducing the average pore size of the through pores 131 provided in the flow rate control unit 13, because of such a flow rate control unit 13, the rate of flow of a liquid in the first capturing unit 11 can be made smaller than in the case where the flow rate control unit 13 is not provided.

### <Liquid Holding Unit>

The liquid holding unit 17 is a component for holding a liquid that has passed through the flow rate control unit 13. In addition, the liquid holding unit 17 also functions as a pump for moving the liquid introduced into the liquid introduction unit 14 toward the downstream side. As long as the liquid holding unit 17 has these functions, the specific configuration thereof is not particularly limited and may be suitably employed from known materials. For example, a material such as a nitrocellulose membrane, a filter paper, or a glass-fiber filter paper may be employed as the liquid holding unit 17.

The target substance measurement kit according to the present disclosure is suitable as an immunochromatography measurement kit for detecting a target substance by immunochromatography.

### (2) Target Substance Measurement Device

Next, each component of the target substance measurement device D1 will be described. The target substance measurement device D1 includes at least a light irradiation unit 21 and a light detection unit 22 (see Fig. 1 again).

### <Light Irradiation Unit>

The light irradiation unit 21 is a component for irradiating the first capturing unit 11 of the target substance measurement kit K1 described above with light (see Fig. 1, arrow L1). As long as color development or light emission originating from a labeled substance in a complex of a target substance and a detection reagent captured by the first capturing unit 11 can be detected by the light detection unit 22 described below, the specific configuration of the light irradiation unit 21 is not particularly limited. For example, as the light irradiation unit 21, a known light source such as a mercury lamp, a halogen lamp, a xenon lamp, a LED source, or a laser light source may be employed.

### <Light Detection Unit>

The light detection unit 22 is a component for detecting light emitted from the first capturing unit 11 (see Fig. 1, arrow L2). As long as the light detection unit 22 can detect color development or light emission originating from a labeled substance, the specific configuration thereof is not particularly limited. For example, as the light detection unit 22, an area image sensor or a photomultiplier tube, such as a CCD or CMOS device, or the like may be employed.

In the target substance measurement device D1 described above, in addition to the light irradiation unit 21 and the light detection unit 22, components necessary for irradiation with the light L1 and reception of the light L2, such as an optical path switching mechanism 23 composed of a dichroic mirror and the like and a lens for concentrating the lights L1 and L2 (in Fig. 1, a lens is not shown), may be suitably employed. In addition, the target substance measurement device D1 may also include, for example, a component that irradiates the second capturing unit 12 described above with light and a component that detects light emitted from the second capturing unit 12. In addition, the light irradiation unit 21 and the light detection unit 22 may also be configured to irradiate each of the first capturing unit 11 and the second capturing unit 12 with the light L1 and detect light emission or color development from each unit. Further, the target substance measurement device D1 may also include a control unit that controls, for example, the timing of the irradiation of the first capturing unit 11 with the light L1 by the light irradiation unit 21.

Incidentally, with respect to the target substance measurement kit K1 described above, by selecting the labeled substance used as a detection reagent and the like, the detection of a target substance may also be performed by visual judgement.

### (3) Detection of Target Substance by Target Substance Measurement System

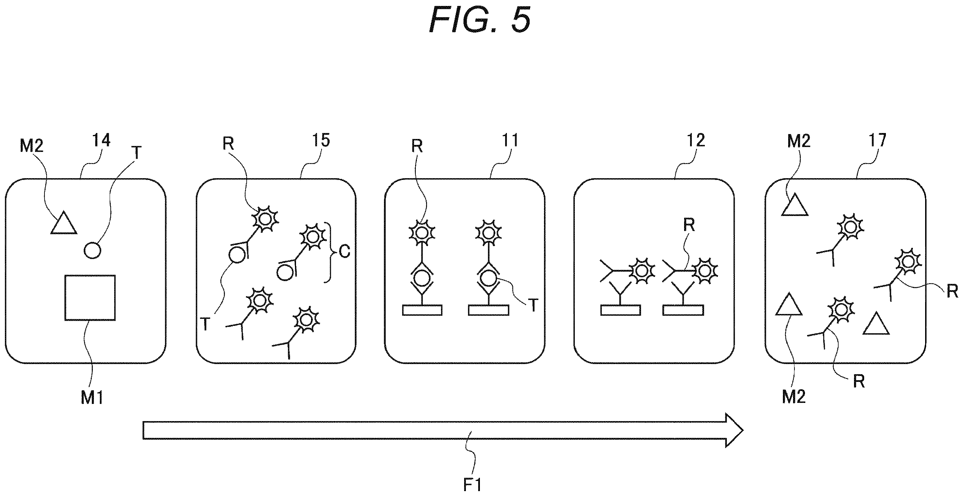

The detection of a target substance by the target substance measurement system S1 will be described with reference to Fig. 5. Fig. 5 is a schematic diagram showing the detection of a target substance by a target substance measurement system. The figure schematically shows, with respect to a target substance T and the like contained in a liquid introduced into the liquid introduction unit 14, their behavior and the like in each unit.

A target substance T in a liquid introduced into the liquid introduction unit 14 moves toward the reagent holding unit 15 (see Fig. 5, arrow F1). At this time, in the liquid introduction unit 14, some impurities M1 can be removed by ultrafiltration. The target substance T that has reached the reagent holding unit 15 binds to a detection reagent R and forms a complex C.

The complex C and the detection reagent R further move toward the downstream side. The complex C is captured by the first capturing unit 11, whereby the complex C turns into an aggregate state. Then, from the first capturing unit 11 irradiated with light by the light irradiation unit 21 of the target substance measurement device D1, light originating from a labeled substance is emitted. The light detection unit 22 of the target substance measurement device D1 detects the emitted light, whereby the target substance T can be detected (in Fig. 5, the target substance measurement device D1 is not shown).

Meanwhile, a detection reagent R that has not bound to the target substance T is captured by the second capturing unit 12, whereby the detection reagent R turns into an aggregate state. As a result, in the second capturing unit 12, color development or light emission from a substance labeled with the detection reagent R can be detected. Accordingly, in the target substance measurement kit K1 including the second capturing unit 12, by measuring color development or light emission in the second capturing unit 12, the success or failure of the implementation of the target substance method by the target substance measurement kit K1 can be judged. In addition, impurities M2 and the like that have not been captured by the first capturing unit 11 and the second capturing unit 12 reach the liquid holding unit 17 and are held in the liquid holding unit 17.

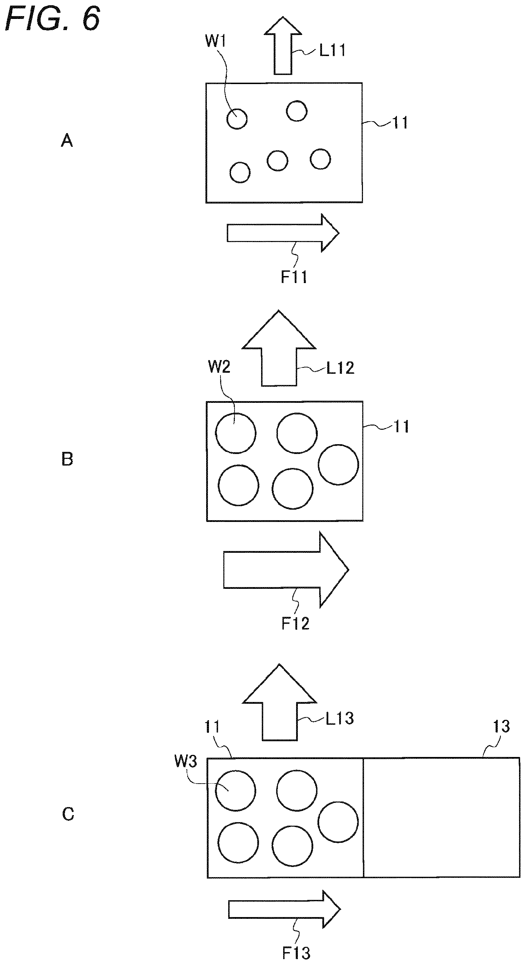

As described above, in the target substance measurement system S1, as a result of the aggregation of the complex C in the first capturing unit 11, the detection of a target substance can be performed. Figs. 6A to 6C are schematic diagram showing the relation between the average pore size of a portion of the first capturing unit 11 through which a liquid flows and the detection of a target substance.

As shown in Fig. 6A, in the target substance measurement kit K1, as a result of the movement of the liquid in the downstream direction (see arrow F11), the complex C aggregates in the first capturing unit 11, and light originating from a labeled substance (see arrow L11) is generated (in Figs. 6A to 6C, the complex is not shown). In this case, when the average pore size of the portion W1 of the first capturing unit 11 through which the liquid flows is small, in the first capturing unit 11, the density of the member forming the first capturing unit 11 is high. As a result, the intensity of light originating from a labeled substance, which is emitted from the first capturing unit 11 having small gaps to the outside of the target substance measurement kit K1, is low.

In contrast, as shown in Fig. 6B, when the average pore size of the portion W2 of the first capturing unit 11 through which the liquid flows is larger than in the case shown in Fig. 6A, the density of the member forming the first capturing unit 11 is low. As a result, the intensity of light originating from a labeled substance, which is emitted from the first capturing unit 11 having increased gaps to the outside of the target substance measurement kit K1, can be increased. However, when the average pore size of the portion W2 of the first capturing unit 11 through which the liquid flows increases, the rate of flow in the first capturing unit 11 rises (Fig. 6B, arrow F12), whereby the efficiency of the capture of the complex in the first capturing unit 11 may decrease.

Thus, as shown in Fig. 6C, in the target substance measurement kit K1 according to this embodiment, the flow rate control unit 13 controls the rate of flow of the liquid in the first capturing unit 11. Accordingly, even in the case where the average pore size of the portion W3 of the first capturing unit 11 through which the liquid flows is increased, a rise in the rate of flow of the liquid in the first capturing unit 11 can be suppressed (Fig. 6C, arrow F13). As a result, without a decrease in the efficiency of the capture of the complex in the first capturing unit 11, the intensity of light originating from a labeled substance, which is emitted from the first capturing unit 11 to the outside of the target substance measurement kit K1, can be increased.

In the target substance measurement system according to this embodiment, the flow rate control unit described above is provided downstream from the first capturing unit. As a result, the rate of flow of a liquid in the first capturing unit can be controlled. Accordingly, even when the average pore size of the portion of the first capturing unit though which the liquid flows is designed to have a more suitable size for the detection of a target substance, a decrease in the efficiency of the capture of the target substance in the first capturing unit can be suppressed. Accordingly, in the target substance measurement system according to this embodiment, a target substance can be detected more sensitively.

In addition, in the target substance measurement system according to this embodiment, use of additional reagents or complicated operation is not required in order to enhance the detection sensitivity. Accordingly, in the target substance measurement system according to this embodiment, a target substance can be detected sensitively in a convenient manner.

The target substance measurement system according the present disclosure is suitable as an immunochromatography measurement system for detecting a target substance by immunochromatography.

### 2. Second Embodiment

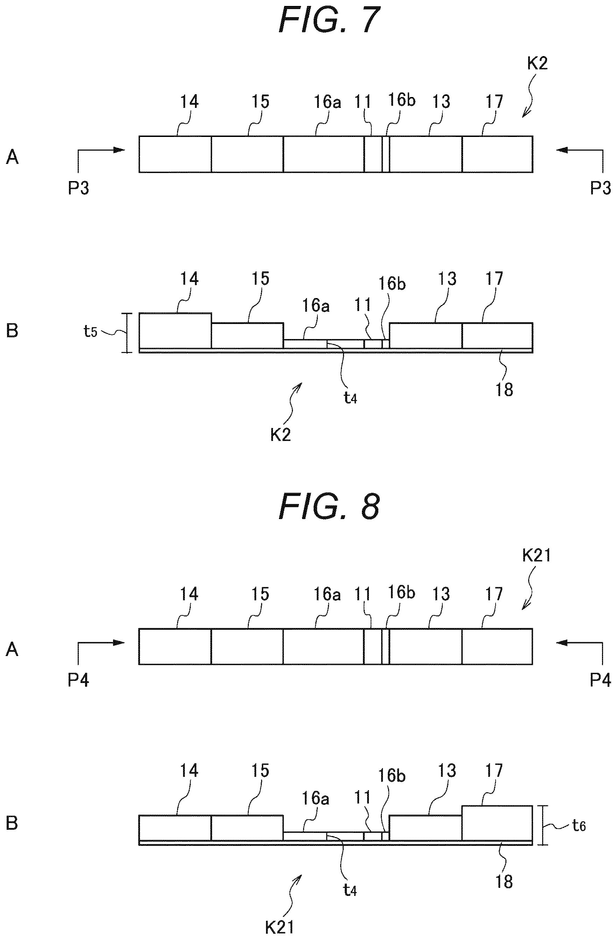

Figs. 7A and 7B schematically show a target substance measurement kit K2 in a target substance measurement system according to a second embodiment of the present disclosure. Fig. 7A is a plan view of the target substance measurement kit K2, and Fig. 7B is a cross-sectional view along the line P3-P3 shown in Fig. 7A.

In this embodiment, the configuration of the target substance measurement device is the same as in the first embodiment described above, and thus the description thereof will be omitted. In addition, of the components of the target substance measurement kit K2, the same components as in the first embodiment described above are indicated with the same reference numerals, and the description thereof will be omitted.

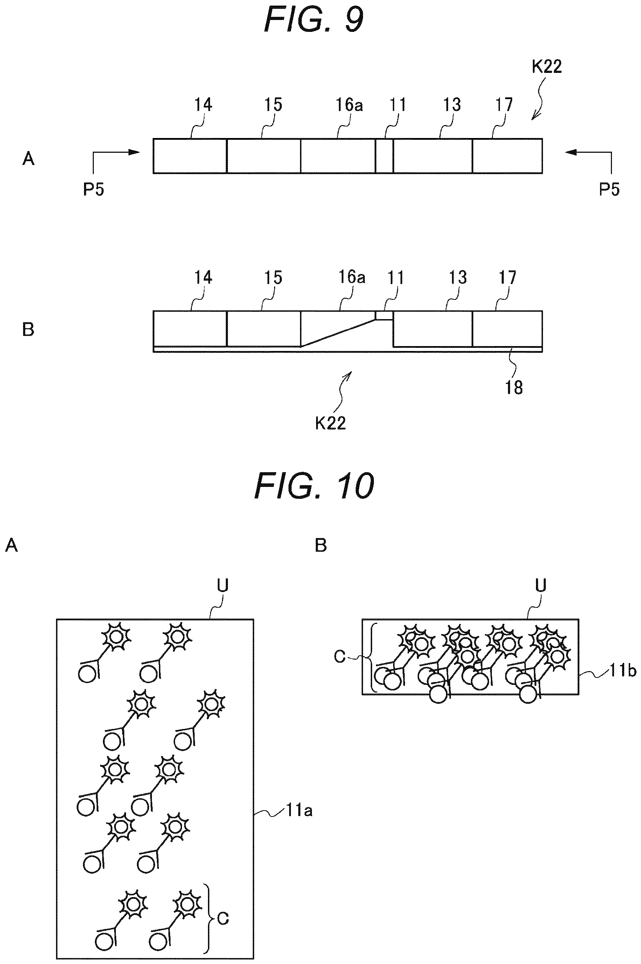

As shown in Fig. 7B, in the target substance measurement kit K2, the thickness of the first capturing unit 11 in the direction transverse to the flow direction of a liquid (see Fig. 7B, t4) is smaller than the thickness of the liquid introduction unit 14 in the direction transverse to the flow direction of the liquid (see Fig. 7B, t5). Incidentally, although the thickness of each of the reagent holding unit 15, the development units 16a and 16b, the flow rate control unit 13, and the liquid holding unit 17 shown in Fig. 7B is the same in Figs. 7A and 7B, the thicknesses of the units may also be different. This also applies to the target substance measurement kits K21 and K22 shown in Figs. 8A and 8B and Figs. 9A and 9B described below.

Figs. 8A and 8B schematically show another configuration example of the target substance measurement kit according to this embodiment. Fig. 8A is a plan view of a target substance measurement kit K21, and Fig. 8B is a cross-sectional view along the line P4-P4 shown in Fig. 8A. As shown in Fig. 8B, the thickness of the first capturing unit 11 in the direction transverse to the flow direction of a liquid (see Fig. 8B, t4) is smaller than the thickness of the liquid holding unit 17 in the direction transverse to the flow direction of the liquid (see Fig. 8B, t6).

In addition, in the target substance measurement kits shown in Figs. 7A and 7B and Figs. 8A and 8B, in addition to the first capturing unit 11, the development units 16a and 16b are also formed thin. However, in this embodiment, at least the first capturing unit 11 should be formed thin, and the configuration is not limited to those shown in Figs. 7A and 7B and Figs. 8A and 8B.

Figs. 9A and 9B are schematic diagrams showing a configuration example of the target substance measurement kit according to this embodiment. Fig. 9A is a plan view of a target substance measurement kit K22, and Fig. 9B is a cross-sectional view along the line P5-P5 shown in Fig. 9A. As shown in Fig. 9B, in this embodiment, it is also possible that only the first capturing unit 11 is formed thin, and the development unit 16a in contact with the first capturing unit 11 is configured such that the thickness gradually decreases toward the downstream side.

Figs. 10A and 10B show the relation between the thickness of the first capturing unit 11 and the detection of a target substance. Figs. 10A and 10B schematically show the first capturing unit 11 in a target substance measurement kit. A first capturing unit 11b shown in Fig. 10B is formed thinner than a first capturing unit 11a shown in Fig. 10A. Therefore, in the case where the amount of the complex C present in the first capturing unit 11 is constant, in the first capturing unit 11b, the density of the complex C is higher in a region closer to the surface U.

In the first capturing unit 11, even when the complex C is in an aggregate state, in the case where the complex C is present in a deep position far from the surface U, due to the member forming the first capturing unit 11, it may be difficult for color development or light emission originating from a labeled substance in the complex to reach the outside of the target substance measurement kit. In contrast, in the target substance measurement kits K2, K21, and K22 according to this embodiment, because the first capturing unit 11 is formed thin, the density of the complex C increases, and also the complex C aggregates in a shallow position from the surface U of the first capturing unit 11. As a result, the intensity of color development or light emission originating from a labeled substance can be enhanced.

It is preferable that the thickness of the first capturing unit 11 (see Fig. 7B, t4) is 0.01 mm or more and 0.10 mm or less. When the thickness of the first capturing unit 11 is 0.10 mm or less, the intensity of light emission from a labeled substance in the first capturing unit 11 can be enhanced. In addition, when the first capturing unit 11 is 0.01 mm or more, the strength of the first capturing unit 11 itself can be sufficiently obtained. In addition, when the first capturing unit 11 is provided on the substrate 18, the strength of the first capturing unit 11 can be further enhanced.

In the target substance measurement system according to this embodiment, the first capturing unit 11 is formed thinner as compared with the other components. Therefore, the complex C can be present at high density near the surface of the first capturing unit 11, thereby enhancing the intensity of color development or light emission from a labeled substance. Accordingly, the target substance can be measured more sensitively.

For example, according to the immunochromatography measurement method disclosed in Patent Document 2, the support substance at the reactive site is compressed to reduce the thickness. However, in this case, the density of the support substance also increases, whereby gaps in the support substance decrease. As a result, the light emission signal from the labeled substance may be rather reduced. In contrast, in the target substance measurement system according to this embodiment, because the first capturing unit 11 is formed thin, the labeled substance can be measured without a decrease in gaps or the resulting decrease in the intensity of color development or light emission from the labeled substance.

Other effects of the target substance measurement system according to the second embodiment are similar to those of the target substance measurement system according to the first embodiment described above.

### 3. Third Embodiment

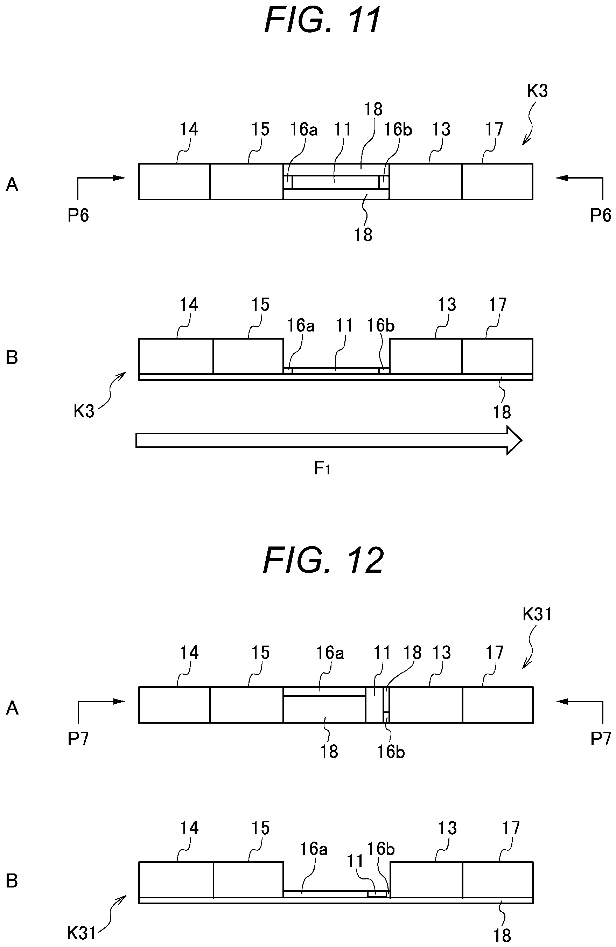

Figs. 11A and 11B schematically show a target substance measurement kit K3 in a target substance measurement system according to a third embodiment of the present disclosure. In addition, in this embodiment, the configuration of the target substance measurement device is the same as in the first embodiment described above, and thus the description thereof will be omitted. Further, of the components of the target substance measurement kit K3, the same components as in the first embodiment described above are indicated with the same reference numerals, and the description thereof will be omitted.

Fig. 11A is a plan view of a target substance kit K3, and Fig. 11B is a cross-sectional view along the line P6-P6 shown in Fig. 11A. As shown in Fig. 11A, in the target substance measurement kit, the longitudinal direction of the first capturing unit 11 extends in the flow direction of the liquid (arrow F1).

The configuration in which the longitudinal direction of the first capturing unit 11 extends in the flow direction is not limited to the configuration of the first capturing unit 11 shown in Figs. 11A and 11B. Figs. 12A and 12B are schematic diagrams showing a configuration example of the target substance measurement kit according to the present disclosure. Fig. 12A is a plan view of a target substance measurement kit K31, and Fig. 12B is a cross-sectional view along the line P7-P7 shown in Fig. 12A. For example, as shown in Fig. 12A, the configuration may be such that the liquid flow direction changes at the connection between the development units 16a and 16b and the first capturing unit 11.

In the target substance measurement system according to this embodiment, the longitudinal direction of the first capturing unit 11 provided in the target substance measurement kit extends in the flow direction of a liquid. Accordingly, the time taken for a liquid containing a complex to pass through the first capturing unit 11 is prolonged, making it possible to enhance the efficiency of the capture of the complex by the first capturing unit. As a result, the amount of complex captured by the first capturing unit 11 can be increased, and the target substance detection sensitivity can be improved. Other effects of the target substance measurement system according to the third embodiment are similar to those of the target substance measurement system according to the first embodiment described above.

### 4. Fourth Embodiment

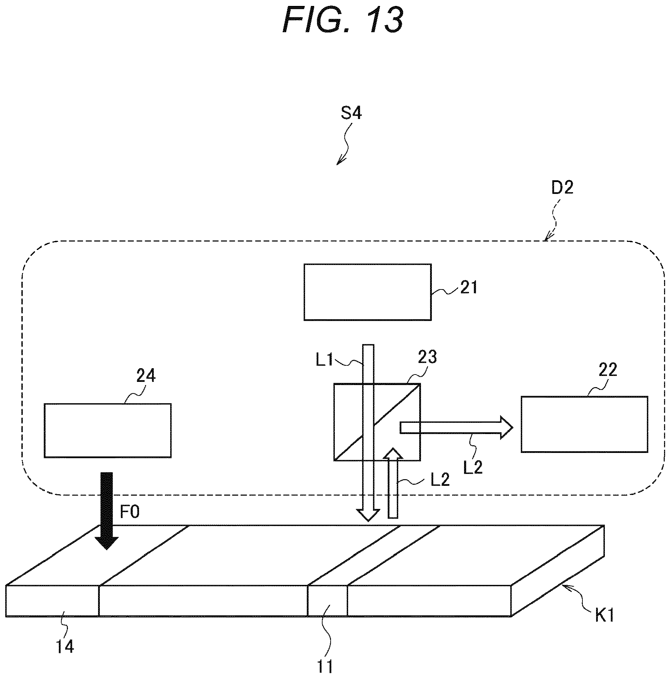

Fig. 13 schematically shows a configuration example of a target substance measurement system according to a fourth embodiment of the present disclosure. In the target substance measurement system S4 according to this embodiment, the same components as in the target substance measurement system S1 according to the first embodiment described above are indicated with the same reference numerals, and the description thereof will be omitted.

As shown in Fig. 13, the target substance measurement device D2 includes a liquid injection mechanism 24 that injects a liquid into the liquid introduction unit 14 of the target substance measurement kit K1 (see arrow F0). As long as the liquid injection mechanism 24 can introduce the liquid described above into the liquid introduction unit 14, the specific configuration thereof is not particularly limited and may be suitably employed from the configurations of known liquid injection devices and the like according to the properties of the liquid and the like.

In addition, the liquid injection mechanism 24 may include a component for injecting another liquid different from the liquid containing a target substance into the target substance measurement kit K1. Another liquid is, for example, a liquid for causing light emission from a labeled substance that binds to a detection reagent. Specific examples thereof are reagent solutions containing hydrogen peroxide, peroxidase, alkaline phosphatase, luciferase, and the like. After introducing the liquid containing a target substance, such a reagent solution is introduced into the target substance measurement kit K1. As a result, without requiring light irradiation by the light irradiation unit 21 described above, the target substance can be measured using light emission from the labeled substance utilizing chemiluminescence, bioluminescence, or the like.

In the target substance measurement system according to this embodiment, because of the presence of the liquid injection mechanism, it is not necessary to manually introduce a liquid into the target substance measurement kit. Accordingly, accidents in that the user accidentally touches the liquid can be prevented. Other effects of the target substance measurement system according to the fourth embodiment are similar to those of the target substance measurement system according to the first embodiment described above.

Incidentally, the effects described above are merely illustrative and not restrictive, and there may also be other effects.

The present disclosure may also be configured as follows.
(1) A target substance measurement kit including: a capturing unit that captures a target substance contained in a liquid; and a flow rate control unit that controls the rate of flow of the liquid in the capturing unit, the flow rate control unit being provided downstream from the capturing unit in the flow direction of the liquid.
(2) The target substance measurement kit according to (1), further including, upstream from the capturing unit in the flow direction, a liquid introduction unit into which the liquid is introduced, wherein the thickness of the capturing unit in the direction transverse to the flow direction is smaller than the thickness of the liquid introduction unit in the transverse direction.
(3) The target substance measurement kit according to (1), further including, downstream from the flow rate control unit in the flow direction, a liquid holding unit that absorbs the liquid, wherein the thickness of the capturing unit in the direction transverse to the flow direction is smaller than the thickness of the liquid holding unit in the transverse direction.
(4) The target substance measurement kit according to any of (1) to (3), wherein the thickness of the capturing unit in the direction transverse to the flow direction is 0.01 mm or more and 0.10 mm or less.
(5) The target substance measurement kit according to any of (1) to (4), wherein the longitudinal direction of the capturing unit extends in the flow direction.
(6) The target substance measurement kit according to any of (1) to (5), wherein the flow rate control unit includes a porous body, and the average pore size of the porous body is smaller than the average pore size of a portion of the capturing unit through which the liquid flows.
(7) The target substance measurement kit according to any of (1) to (5), wherein the thickness of the flow rate control unit in the direction transverse to the flow direction is smaller than the thickness of the capturing unit in the transverse direction.
(8) The target substance measurement kit according to any of (1) to (5), wherein the flow rate control unit includes a filter having through pores formed therein, and the average pore size of the through pores is smaller than the average pore size of a portion of the capturing unit through which the liquid flows.
(9) A target substance measurement system including a target substance measurement kit and a target substance measurement device, the target substance measurement kit including: a capturing unit that captures a target substance contained in a liquid; and a flow rate control unit that controls the rate of flow of the liquid in the capturing unit, the flow rate control unit being provided downstream from the capturing unit in the flow direction of the liquid, the target substance measurement device including: a light irradiation unit that irradiates the capturing unit with light; and a light detection unit that detects light emitted from the capturing unit.
(10) The target substance measurement system according to (9), wherein the target substance measurement kit includes, upstream from the capturing unit in the flow direction, a liquid introduction unit into which the liquid is introduced, and the target substance measurement device includes a liquid injection mechanism that injects the liquid into the liquid introduction unit.
(11) An immunochromatography measurement kit including: a capturing unit containing an antibody immobilized on a flow path through which a liquid containing a target substance flows; and a flow rate control unit containing a porous body disposed on the flow path, the flow rate control unit being provided downstream from the capturing unit in the flow direction of the liquid.
(12) An immunochromatography measurement system including an immunochromatography measurement kit and a target substance measurement device, the immunochromatography measurement kit including: a capturing unit containing an antibody immobilized on a flow path through which a liquid containing a target substance flows; and a flow rate control unit containing a porous body disposed on the flow path, the flow rate control unit being provided downstream from the capturing unit in the flow direction of the liquid, the target substance measurement device including: a light irradiation unit that irradiates the capturing unit with light; and a light detection unit that detects light emitted from the capturing unit.

### REFERENCE SIGNS LIST

- C:: Complex
- D1, D2:: Target substance measurement device
- K1, K11, K2, K21, K22, K3, K31:: Target substance measurement kit
- M1, M2:: Impurity
- R:: Detection reagent
- S1, S4:: Target substance measurement system
- T:: Target substance
- 11, 11a, 11b:: Capturing unit (first capturing unit)
- 12:: Second capturing unit
- 13:: Flow rate control unit
- 131:: Through pore
- 14:: Liquid introduction unit
- 15:: Reagent holding unit
- 16a, 16b, 16c:: Development unit
- 17:: Liquid holding unit
- 18:: Substrate
- 21:: Light irradiation unit
- 22:: Light detection unit
- 23:: Optical path switching mechanism
- 24:: Liquid injection mechanism

## Claims

1. A target substance measurement kit comprising:
a capturing unit that captures a target substance contained in a liquid; and
a flow rate control unit that controls the rate of flow of the liquid in the capturing unit,
the flow rate control unit being provided downstream from the capturing unit in the flow direction of the liquid.

2. The target substance measurement kit according to claim 1, further comprising, upstream from the capturing unit in the flow direction, a liquid introduction unit into which the liquid is introduced, wherein
the thickness of the capturing unit in the direction transverse to the flow direction is smaller than the thickness of the liquid introduction unit in the transverse direction.

3. The target substance measurement kit according to claim 1, further comprising, downstream from the flow rate control unit in the flow direction, a liquid holding unit that absorbs the liquid, wherein
the thickness of the capturing unit in the direction transverse to the flow direction is smaller than the thickness of the liquid holding unit in the transverse direction.

4. The target substance measurement kit according to claim 2, wherein the thickness of the capturing unit in the direction transverse to the flow direction is 0.01 mm or more and 0.10 mm or less.

5. The target substance measurement kit according to claim 2, wherein the longitudinal direction of the capturing unit extends in the flow direction.

6. The target substance measurement kit according to claim 1, wherein
the flow rate control unit includes a porous body, and
the average pore size of the porous body is smaller than the average pore size of a portion of the capturing unit through which the liquid flows.

7. The target substance measurement kit according to claim 1, wherein the thickness of the flow rate control unit in the direction transverse to the flow direction is smaller than the thickness of the capturing unit in the transverse direction.

8. The target substance measurement kit according to claim 1, wherein
the flow rate control unit includes a filter having through pores formed therein, and
the average pore size of the through pores is smaller than the average pore size of a portion of the capturing unit through which the liquid flows.

9. A targetsubstance measurementsystem comprising a target substance measurement kit and a target substance measurement device,
the target substance measurement kit including:
a capturing unit that captures a target substance contained in a liquid; and
a flow rate control unit that controls the rate of flow of the liquid in the capturing unit,
the flow rate control unit being provided downstream from the capturing unit in the flow direction of the liquid,
the target substance measurement device including:
a light irradiation unit that irradiates the capturing unit with light; and
a light detection unit that detects light emitted from the capturing unit.

10. The target substance measurement system according to claim 9, wherein
the target substance measurement kit includes, upstream from the capturing unit in the flow direction, a liquid introduction unit into which the liquid is introduced, and
the target substance measurement device includes a liquid injection mechanism that injects the liquid into the liquid introduction unit.

11. An immunochromatography measurement kit comprising:
a capturing unit containing an antibody immobilized on a flow path through which a liquid containing a target substance flows; and
a flow rate control unit containing a porous body disposed on the flow path,
the flow rate control unit being provided downstream from the capturing unit in the flow direction of the liquid.

12. An immunochromatography measurement system comprising an immunochromatography measurement kit and a target substance measurement device,
the immunochromatography measurement kit including:
a capturing unit containing an antibody immobilized on a flow path through which a liquid containing a target substance flows; and
a flow rate control unit containing a porous body disposed on the flow path,
the flow rate control unit being provided downstream from the capturing unit in the flow direction of the liquid,
the target substance measurement device including:
a light irradiation unit that irradiates the capturing unit with light; and
a light detection unit that detects light emitted from the capturing unit.
